(19) Europäisches Patentamt
European Patent Office
Office européen des brevets

(11) Veröffentlichungsnummer: **0 045 386**
**B1**

# (12) EUROPÄISCHE PATENTSCHRIFT

(45) Veröffentlichungstag der Patentschrift: **07.03.84**

(51) Int. Cl.³: **C 07 C 121/44**

(21) Anmeldenummer: **81105300.8**

(22) Anmeldetag: **08.07.81**

## (54) Verfahren zur Herstellung von Aminonitrilen.

(30) Priorität: **01.08.80 DE 3029205**

(43) Veröffentlichungstag der Anmeldung: **10.02.82 Patentblatt 82/6**

(45) Bekanntmachung des Hinweises auf die Patenterteilung: **07.03.84 Patentblatt 84/10**

(84) Benannte Vertragsstaaten: **BE CH DE FR GB LI SE**

(56) Entgegenhaltungen:
**FR - A - 1 448 743**
**FR - A - 2 299 315**
**US - A - 3 546 271**

(73) Patentinhaber: **BASF Aktiengesellschaft, Carl-Bosch-Strasse 38, D-6700 Ludwigshafen (DE)**

(72) Erfinder: **Distler, Harry, Dr., In den Hahndornen 5, D-6719 Bobenheim (DE)**
Erfinder: **Hock, Karl-Ludwig, Dr., Hillesheimer Strasse 3, D-6700 Ludwigshafen (DE)**

EP 0 045 386 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Aminonitrilen

Die Erfindung betrifft ein Verfahren zur Herstellung von Aminonitrilen durch Umsetzung von Stickstoffverbindungen mit Formaldehyd und Blausäure in Gegenwart zusätzlicher Säure bei einem pH unterhalb 2 bei einer Temperatur von 10 bis 70 °C umsetzt, wobei die Konzentration der Blausäure während der Reaktion nicht mehr als 3 Gewichtsprozent, bezogen auf das Reaktionsgemisch, beträgt.

Es ist bekannt, dass man Ammoniak, Blausäure und Formaldehyd, gegebenenfalls in Gegenwart einer zusätzlichen Säure, bei erhöhter Temperatur, z.B. von 40 bis 60 °C, in exothermer Reaktion zu Nitrilotriacetonitril umsetzen kann (DE-AS 14 93 910; US-PS 2 855 428; US-PS 3 959 342). Die Gesamtausbeute ist bei diesen raschen exothermen Umsetzungen unbefriedigend; auch Erniedrigung der Reaktionstemperatur verhindern die Bildung von unerwünschten Nebenprodukten nur in geringem Masse, während andererseits eine Erhöhung der Reaktionszeit notwendig wird und damit die Raum-Zeit-Ausbeute absinkt. Ein entsprechendes Verfahren zeigt auch die US-Patentschrift 3 984 453.

Die Blausäurekonzentration liegt während der Reaktion, entsprechend den Beispielen der deutschen Auslegeschrift 14 93 910 (Beispiel 1), bei 10 bis 20,3 Gewichtsprozent, entsprechend denen der US-Patentschrift 2 855 428 bei 10 bis 30,7 Gewichtsprozent und entsprechend denen der US-Patentschrift 3 959 342 bei 7 bis 12 Gewichtsprozent, bezogen auf das Reaktionsgemisch.

Zur Vermeidung dieser Schwierigkeiten lehrt die deutsche Offenlegungsschrift 17 68 257 einen Zweistufenprozess, bei dem man zunächst eine Mischung von Ammoniak, Cyanwasserstoff und Formaldehyd in einem sauren, wässrigen Medium bei einer Temperatur von etwa 0 bis 45 °C zu einem Methylenbisiminodiacetonitril enthaltenden Reaktionsgemisch als Zwischenprodukt umsetzt und anschliessend die als Zwischenprodukt erhaltene Reaktionsmischung auf eine Temperatur von etwa 45 bis 120 °C erhitzt. Das pH des Reaktionsgemischs soll unterhalb 3 liegen. Im Ausgangsgemisch beträgt im allgemeinen das Molverhältnis von Ammoniak zu Cyanwasserstoff zu Formaldehyd zwischen etwa 1:3:3 und etwa 1:4:4. Wie die Beispiele zeigen, beträgt die Blausäurekonzentration von Anfang an und während der ersten Stufe bei 0 bis 45 °C mindestens 7, im allgemeinen zwischen 7 und 20 Gewichtsprozent, während der zweiten Stufe der Umsetzung bei 45 bis 120 °C mindestens 7, im allgemeinen bis 15 Gewichtsprozent, bezogen auf das Reaktionsgemisch. Ausbeute und Reinheit des so erhaltenen Nitrilotriacetonitril sind trotz des umständlichen 2-Stufen-Prozesses unbefriedigend.

Die hohen Reinheitsforderungen, die heute an die Reinheit von Aminoessigsäuren, insbesondere der Nitrilotriessigsäure, d.h. der Verseifungsprodukte der Endstoffe I, auf dem Waschmittelsektor gestellt werden, zwingen zu einer hohen Reinheit der Endstoffe I; eine gaschromatographische Reinheit von mindestens 99% soll erreicht werden. Insbesondere sollte der Gehalt an Iminodiessigsäure nicht grösser als 0,2% sein. Bei den bekannten einstufigen Verfahren werden häufig Verunreinigungen, z.B. des Aminotriacetonitrils mit Iminodiacetonitril von 0,2 bis 2 Gewichtsprozent, bezogen auf Aminotriacetonitril, mit Methylenbisaminoverbindungen von 0,1 bis 2 Gewichtsprozent, bezogen auf Aminotriacetonitril, mit Cyanhydrin von 0,01 bis 0,1 Gewichtsprozent, bezogen auf Aminotriacetonitril, mit Polyoxymethylenen von 0,01 bis 0,1 Gewichtsprozent, bezogen auf Aminotriacetonitril, mit α-Aminomonoacetonitril, und mit sonstigen Nebenstoffen von 0,1 bis 0,2 Gewichtsprozent, bezogen auf Aminotriacetonitril, beobachtet. Bei vorgenanntem Zweistufenprozess beobachtet man in der Regel Verunreinigungen des Aminotriacetonitrils mit Iminodiacetonitril von 0,2 bis 0,8 Gewichtsprozent, bezogen auf Aminotriacetonitril, mit Methylenbisaminoverbindungen von 0,2 bis 0,5 Gewichtsprozent, bezogen auf Aminotriacetonitril, mit Cyanhydrin von 0,1 bis 0,2 Gewichtsprozent, bezogen auf Aminotriacetonitril, mit Polyoxymethylenen von 0,01 bis 0,1 Gewichtsprozent, bezogen auf Aminotriacetonitril, mit α-Aminomonoacetonitril von 0,1 bis 0,2 Gewichtsprozent, bezogen auf Aminotriacetonitril, und mit sonstigen Nebenstoffen von ca 0,2 Gewichtsprozent, bezogen auf Aminotriacetonitril. Da Methylenbisiminodiacetonitril kristallin anfällt und in fester Form, d.h. in einer Art topochemischer Reaktion, mit Blausäure und Formaldehyd weiter zu Nitrilotriacetonitril umgesetzt werden muss, sind Verunreinigungen im Endstoff nicht auszuschliessen.

Es ist aus der deutschen Patentschrift 25 03 582 bekannt, dass man N-Alkylglycinnitrile durch Umsetzung von Formaldehyd mit primären oder sekundären N-Alkylaminen und Blausäure in Gegenwart von Wasser während 0,1 bis 4 Stunden bei einer Temperatur von 0 bis 40 °C erhält, wobei die Konzentration der Blausäure während der Reaktion nicht mehr als 0,1 Gewichtsprozent, bezogen auf das Reaktionsgemisch, beträgt.

Es wurde nun gefunden, dass man Aminoacetonitrile der Formel

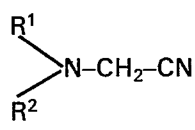

I,

worin $R^1$ und $R^2$ gleich oder verschieden sein können und jeweils den Rest $-CH_2-CN$ oder den Rest

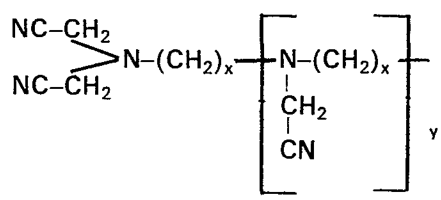

bedeuten, $R^1$ auch für einen aliphatischen Rest steht, die einzelnen Reste x und y gleich oder verschieden sein können und jeweils eine ganze Zahl bezeichnen, y auch für 0 steht, durch Umsetzung von Ammoniak oder Aminen mit Formaldehyd und Blausäure in Gegenwart einer weiteren Säure vorteilhaft erhält, wenn man Stickstoffverbindungen der Formel

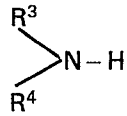
II,

worin $R^3$ und $R^4$ ein Wasserstoffatom bedeuten, $R^3$ und/oder $R^4$ auch, wenn $R^1$ und/oder $R^2$ den Rest

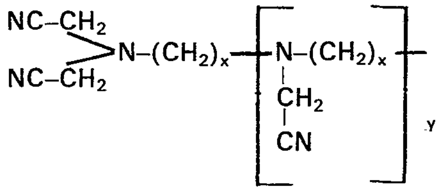
bezeichnen, für den Rest

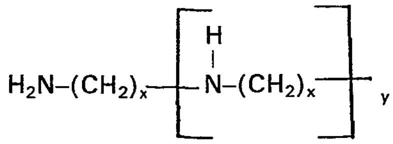
stehen, $R^3$ auch, wenn $R^1$ für

einen aliphatischen Rest steht, die Bedeutung von $R^1$ besitzt, x und y die vorgenannte Bedeutung besitzen, bei einem pH unterhalb 2 bei einer Temperatur von 10 bis 70 °C umsetzt, wobei die Konzentration der Blausäure während der Reaktion nicht mehr als 3 Gewichtsprozent, bezogen auf das Reaktionsgemisch, beträgt.

Die Umsetzung kann für den Fall der Verwendung von Ammoniak durch die folgenden Formeln wiedergegeben werden:

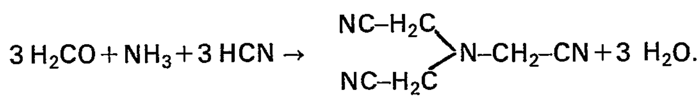

Im Vergleich zu den bekannten Verfahren liefert das Verfahren nach der Erfindung auf einfacherem und wirtschaftlicherem Wege Aminoacetonitrile in besserer Ausbeute und Reinheit. Das Verfahren ist vergleichsweise gerade für den Betrieb im grosstechnischen Massstab und für einen kontinuierlichen Betrieb geeignet, bietet keine wesentlichen Abwässerfragen und ist umweltfreundlicher. Als Nebenprodukte treten im allgemeinen Iminodiacetonitril nur unterhalb 0,2 Gewichtsprozent, Methylenbisaminoverbindungen unterhalb 0,001 Gewichtsprozent, Cyanhydrin unterhalb 0,001 Gewichtsprozent, Polyoxymethylene unterhalb 0,001 Gewichtsprozent, α-Aminomonoacetonitril unterhalb 0,001 Gewichtsprozent, bezogen auf Aminotriacetonitril, auf und eine entsprechende geringere Nebenproduktbildung ist auch bei den weiteren Aminoacetonitrilen zu beobachten. Alle diese vorteilhaften Eigenschaften sind im Hinblick auf den Stand der Technik überraschend. Man hätte gerade durch die niedrige Reaktionstemperatur und Blausäurekonzentration zu Beginn und während der Reaktion mit Bezug auf die Stickstoffverbindung II eine erhöhte Bildung von Methylenbisaminoverbindungen, eine längere Reaktionszeit und entsprechend stärkere Zersetzung und Bildung von Zersetzungsprodukten erwarten müssen. Im Hinblick auf die deutsche Patentschrift 25 03 582 war es überraschend, dass α-Aminomonoacetonitrile und α-Iminodiacetonitrile nicht weit stärker als Nebenprodukte gebildet werden.

Formaldehyd kann in flüssiger Form oder als Gas, im allgemeinen in Form seiner wässrigen, zweckmässig von 10- bis 50-, vorzugsweise von 30- bis 40-gewichtsprozentigen Lösung verwendet werden. Blausäure kommt als Gas oder zweckmässig in flüssiger Form in Betracht. Der Ausgangsstoff II kann allein oder zweckmässig in Lösung, vorzugsweise in wässriger Lösung verwendet werden; zweckmässig sind 40- bis 60-gewichtsprozentige Lösungen. Ammoniak wird vorzugsweise als Formaldehydaddukt eingesetzt, z.B. Hexamethylentetramin, welches durch Zusammengeben der Komponenten bei Raumtemperatur gebildet wird. Dies hat den Vorteil, dass man wesentlich weniger Säure, z.B. die Hälfte bis ein Drittel der stöchiometrischen Menge an Schwefelsäure, für die Neutralisierung des Ammoniaks benötigt und damit auch weniger Salzfracht für die späteren Ablaugen erzeugt.

Man kann die drei Ausgangsstoffe in stöchiometrischer Menge oder jede der Komponenten jeweils im Überschuss umsetzen, vorzugsweise in einer Menge von 1 bis 1,1 Mol Ausgangsstoff II und/oder von 1 bis 1,1 Mol Blausäure je Mol Formaldehyd (bezeichnet 100%). Bevorzugte Ausgangsstoffe II und dementsprechend bevorzugte Endstoffe I sind solche, in deren Formeln $R^3$ und $R^4$ gleich oder verschieden sein können und jeweils ein Wasserstoffatom bezeichnen, $R^3$ und/oder $R^4$ auch für den Rest

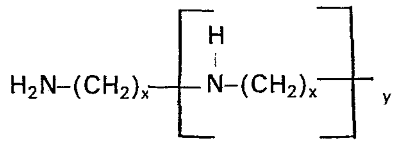 wenn $R^1$ und/oder $R^2$ den Rest

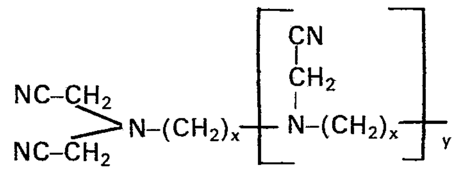 bezeichnen, stehen, $R^1$ und $R^2$ gleich oder verschieden sein können und jeweils den Rest $-CH_2-CN$ oder den Rest

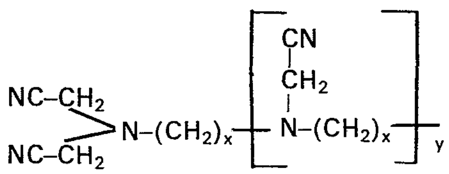

bezeichnen, $R^3$ und $R^1$ darüber hinaus auch einen Alkylrest mit 1 bis 8, insbesondere 1 bis 4 Kohlenstoffatomen, bedeuten, die einzelnen Reste x und y gleich oder verschieden sein können und jeweils x 1 oder 3 oder insbesondere 2 und y 3 oder 2 oder insbesondere 0 oder 1 bezeichnen. Die vorgenannten Reste können noch durch unter den Reaktionsbedingungen inerte Gruppen, z.B. Alkylgruppen, Alkoxygruppen mit jeweils 1 bis 4 Kohlenstoffatomen, substituiert sein. Beträgt y 0, ist x zweckmässig 2 oder grösser als 2, vorteilhaft 2 oder 3.

Es kommen z.B. als Ausgangsstoffe II in Betracht: Ammoniak; Methyl-, Äthyl-, n-Propyl-, Isopropyl-, n-Butyl-, Isobutyl-, sek.-Butyl-, tert.-Butyl-, Pentyl-, Pentyl-(2)-, Pentyl-(3)-, n-Hexyl-, n-Heptyl-, n-Octyl-, n-Nonyl-, n-Decyl-, 2-Äthylhexyl-amin; Äthylen-, Propylen-, Isopropylen-, Butylen-, sek.-Butylen-, Isobutylen-, tert.-Butylen-diamin; Diäthylen-, Dipropylen-, Diisopropylen-, Dibutylen-, Diisobutylen-, Di-sek.-butylen-, Di-tert.-butylen-triamin, Triäthylen-, Tripropylen-, Triisopropylen-, Tributylen, Tri-sek.-butylen-, Triisobutylen-, Tri-tert.-butylen-tetramin, Tetraäthylen-, Tetrapropylen-, Tetraisopropylen-, Tetrabutylen-, Tetraisobutylen-, Tetra-sek.-butylen-, Tetra-tert.-butylen-pentamin; Nitrilo-(triäthylenamin), -(tripropylenamin), -(triisopropylenamin), -(tributylenamin), -(triisobutylenamin), -(tri-sek.-butylen-amin), -(tri-tert.-butylenamin).

Die Umsetzung wird bei einer Temperatur von 0 bis 70, zweckmässig von 10 bis 60 °C, vorzugsweise von 15 bis 55, insbesondere von 20 bis 49 °C, mit Unterdruck oder Überdruck oder drucklos, diskontinuierlich oder vorzugsweise kontinuierlich durchgeführt. Man verwendet in der Regel Wasser, zweckmässig in Gestalt von wässriger Formaldehydlösung und/oder Säurelösungen, daneben bildet sich bei der Reaktion selbst Wasser; insgesamt kommt eine Gesamtmenge von 40 bis 80, vorzugsweise von 70 bis 60 Gewichtsprozent Wasser, bezogen auf Formaldehyd, in Betracht. Blausäure wird dem Ausgangsgemisch vor und während der Reaktion in einer solchen Menge zugegeben, dass die Konzentration während der Reaktion nicht mehr als 3, zweckmässig von 0,01 bis 3, vorteilhaft von 0,01 bis 2, vorzugsweise von 0,01 bis 1, insbesondere von 0,05 bis 1 Prozent, bevorzugt 0,05 bis 0,7 Gewichtsprozent Blausäure, bezogen auf das Reaktionsgemisch, beträgt. Die Reaktionszeit (im kontinuierlichen Betrieb Verweilzeit) beträgt zweckmässig 0,1 bis 12, vorzugsweise 1 bis 6 Stunden. Bevorzugt verwendet man Wasser allein als Lösungsmittel, gegebenenfalls auch unter den Reaktionsbedingungen inerte, organische Lösungsmittel. Als Lösungsmittel kommen z.B. in Frage: aromatische Kohlenwasserstoffe, z.B. Toluol, Benzol, Äthylbenzol, o-, m-, p-Xylol, Isopropylbenzol, Methylnaphthalin; aliphatische oder cycloaliphatische Kohlenwasserstoffe, z.B. Heptan, α-Pinen, Pinan, Nonan, o-, m-, p-Cymol, Benzinfraktionen innerhalb eines Siedepunktintervalls von 70 bis 190 °C, Cyclohexan, Methylcyclohexan, Petroläther, Dekalin, Hexan, Ligroin, 2,2,4-Trimethylpentan, 2,2,3-Trimethylpentan, 2,3,3-Trimethylpentan, Octan; und entsprechende Gemische. Zweckmässig verwendet man das Lösungsmittel in einer Menge von 40 bis 10 000 Gewichtsprozent, vorzugsweise von 50 bis 1 500 Gewichtsprozent, bezogen auf Ausgangsstoff II.

Man setzt bei einem pH unterhalb 2, zweckmässig bei einem pH zwischen 0,1 bis 2, vorzugsweise von 0,1 bis 1 um. Als zusätzliche Säure und zur Einstellung des pH werden starke Säuren verwendet. Unter starken Säuren werden hier organische oder anorganische, unter den Reaktionsbedingungen inerte Säuren mit einem Säureexponent (pKs) von −7 bis +2,16 verstanden: bezüglich der Definition der Säureexponenten bzw. des pKs-Wertes wird auf Ullmanns Encyklopädie der technischen Chemie, Band 15, Seite 2, verwiesen. Geeignet sind beispielsweise konzentrierte Schwefelsäure, zweckmässig 90- bis 98-gewichtsprozentige, Phosphorsäure, zweckmässig 70- bis 90-gewichtsprozentige, Salzsäure, zweckmässig 10- bis 35-gewichtsprozentige, Salpetersäure, zweckmässig 10- bis 30-gewichtsprozentige, Perchlorsäure, zweckmässig 10- bis 40-gewichtsprozentige, Ameisensäure, zweckmässig 10- bis 90-gewichtsprozentige.

Ebenfalls kommen Chlorwasserstoffgas, Borsäure, Sulfonsäuren wie Benzol- und p-Toluolsulfonsäure, Trichloressigsäure oder stark saure Ionenaustauscher, in Betracht. Bevorzugte Säuren sind konzentrierte Salzsäure oder konzentrierte Schwefelsäure oder Phosphorsäure, insbesondere vorgenannter Konzentrationen. Die Säure verwendet man zweckmässig in Mengen von 0,5 bis 1,5, vorzugsweise 0,5 bis 1 Gewichtsteilen Säure oder äquivalente Mengen an sauren Ionenaustauschern je Gewichtsteil Ausgangsstoff II.

Die Reaktion kann wie folgt durchgeführt werden: Ein Gemisch von Formaldehyd, Wasser, Blausäure, Zusatzsäure und Ausgangsstoff II, gegebenenfalls zusammen mit einem organischen Lösungsmittel, wird bei der Reaktionstemperatur gehalten. Man gibt Anteile der Blausäure in das Ausgangsgemisch und während der Reaktion in Portionen bzw. kontinuierlich so zu, dass die vorgenannte Blausäurekonzentration während der gesamten Reaktionszeit eingehalten wird. Die laufende Messung der Blausäurekonzentrationen erfolgt zweckmässig über eine Silber-Kalomelelektrode. Dann wird aus dem Reaktionsgemisch der Endstoff in üblicher Weise, z.B. durch Destillation oder Filtration, isoliert.

Die Verseifung des Endstoffs I erfolgt nach bekannten Methoden, in der Regel in alkalischem Medium, vorzugsweise in wässrigen Lösungen von Eralkali- oder Alkalihydroxiden oder zweckmässig Alkalisalzen wie Natrium- oder Kaliumcarbonat, -bicarbonat, -acetat, -formiat. Bevorzugt sind Natron- und Kalilauge. Man verwendet z.B. 30- bis 40-gewichtsprozentige Gemische des Endstoffs I in solchen Lösungen und verseift zweckmässig bei einem pH von 9 bis 14 und bei einer Temperatur von 30 bis 100 °C während 2 bis 30 Stunden. Beispielsweise gibt man Alkali, Alkalisalze bzw. wässrige Lösungen dieser Stoffe zu dem Gemisch und verseift den Endstoff I im Gemisch in vorgenannter Weise. Vorteilhaft verwendet man 1 bis 2 Äquivalente Alkali bzw. Alkalisalz bei der Verseifung, bezogen auf den isolierten Endstoff I, und 1 bis 6 Äquivalente Alkali bzw. Alkalisalz, bezogen auf den nicht isolierten, im Reaktionsgemisch in vorgenannter Weise enthaltenen Endstoff I. In einer vorteilhaften Arbeitsweise durchläuft das Reaktionsgemisch verschiedene, vorzugsweise 2 oder 3, Verseifungsstufen steigender Temperatur, z.B. die Rührkessel einer Rührkesselkaskade bei 30 bis 40 °C, 60 °C und 100 °C. Bevorzugt wird die Verseifung mit Unterdruck, zweckmässig von 200 bis 800, insbesondere 400 bis 600 mbar durchgeführt. Durch diese stufenweise Verseifung mit Unterdruck werden überraschend Verfärbungen und Ausbeuteverschlechterung vermieden. Aus dem Verseifungsgemisch wird dann das Salz der dem Endstoff I entsprechenden Aminoessigsäure in üblicher Weise, z.B. durch Eindampfen, Kristallisation und Filtration, isoliert.

Die nach dem Verfahren der Erfindung erhältlichen Aminoacetonitrile sind wertvolle Ausgangsstoffe für die Herstellung von Farbstoffen, Fungiciden, Bactericiden, Textilhilfsmitteln, Kautschukhilfsmitteln, Hilfsmitteln auf dem Gebiet der Enthärtung, Kosmetik, in der Fotoindustrie, Papierindustrie und der Galvanotechnik. Bezüglich der Verwendung wird auf die vorgenannten Veröffentlichungen und Ullmanns Encyklopädie der technischen Chemie (4. Auflage), Band 8, Seiten 198 und 199, sowie Band 17, Seiten 339 bis 341, verwiesen. Die in den folgenden Beispielen aufgeführten Teile bedeuten Gewichtsteile.

Beispiel 1

In einem Reaktor werden stündlich 70 Teile Hexamethylentetramin, 300 Teile wässriger, 30-gewichtsprozentiger Formaldehyd, 162 Teile flüssigen Blausäure und 150 Teile 63-gewichtsprozentige Schwefelsäure aus getrennten Zulaufgefässen zudosiert und bei einer Temperatur von 43 °C zur Reaktion gebracht, wobei während der Reaktion die Blausäurekonzentration 1 Gewichtsprozent, bezogen auf das Gesamtgemisch, nicht übersteigt. Die Blausäurekonzentration liegt durchschnittlich bei 0,5 bis 0,9 Gewichtsprozent, bezogen auf das Gesamtgemisch. Während der Reaktion beträgt das pH < 1 und die Reaktionstemperatur 45 °C. Die Verweilzeit beträgt 6 Stunden. Das Reaktionsgemisch wird abgekühlt und filtriert. Man erhält stündlich 255,8 Teile Nitrilotriacetonitril vom Fp 127,5 bis 128,5 °C (95,46% der Theorie) und einer GC-Reinheit (gaschromatographische Reinheit) von > 99,8 Prozent.

Beispiel 2

a) In einem Rührgefäss werden 240 Teile Äthylendiamin und 823 Teile 55-gewichtsprozentige Schwefelsäure unter Kühlung und Rühren bei 20 °C zusammengegeben. Das Gemisch wird mit 800 Teilen Wasser wieder in Lösung gebracht. Bei einem pH-Wert < 1 und 44 °C werden 1 600 Teile wässriger, 37-gewichtsprozentiger Formaldehyd und 432 Teile flüssige Blausäure gleichzeitig zugegeben, wobei während der Reaktion die Blausäurekonzentration ein Gewichtsprozent, bezogen auf das Gesamtgemisch, nicht übersteigt. Die Verweilzeit im Reaktor beträgt 6 Stunden. Die Blausäurekonzentration liegt durchschnittlich bei 0,5 bis 1,2 Gewichtsprozent, bezogen auf das Gesamtgemisch. Während der Reaktion beträgt das pH < 1 und die Reaktionstemperatur 44 °C. Das Reaktionsgemisch wird abgekühlt und filtriert. Man erhält 823 Teile (95% der Theorie) Äthylendiamintetraacetonitril als farblose Kristalle vom Fp 131 bis 132 °C. Die GC-Reinheit beträgt > 99,8 Prozent.

b) (Verwendung)

In einem Rührgefäss werden 108 Teile Äthylendiaminotetraacetonitril, 200 Teile Wasser und 273 Teile 30-gewichtsprozentige, wässrige NaOH vorgelegt und im Verlauf von 9 Minuten bei 400 mbar zur Reaktion gebracht. Bei einer Verweilzeit von 60 Minuten und 60 °C tritt völlige Lösung ein. Danach wird unter gleichzeitigem Einleiten von Wasserdampf bei 400 mbar während 30 Minuten auf 100 °C erwärmt. Nach einer

Verweilzeit von einer Stunde bei 100 °C ist die Hydrolyse beendet. Man erhält 465 Teile einer fast farblosen Lösung (APHA = 60 bis 80).

Beispiel 3

In einem Rührgefäss werden 337 Teile Diäthylentriamin und 400 Teile Wasser bei 20 °C vorgelegt. In die klare, farblose Lösung werden dann 900 Teile 55-gewichtsprozentige Schwefelsäure unter Kühlung und Rühren bei 20 °C eingebracht. Bei einem pH-Wert $<1$ und 16 °C werden 1 500 Teile wässriger, 30-gewichtsprozentiger Formaldehyd und 405 Teile flüssige Blausäure langsam zugegeben, wobei während der Reaktion die Blausäurekonzentration 2 Gewichtsprozent, bezogen auf das Gesamtgemisch, nicht übersteigt. Die Verweilzeit im Reaktor beträgt 2 Stunden. Die Blausäurekonzentration liegt durchschnittlich bei 0,5 bis 1 Gewichtsprozent, bezogen auf das Gesamtgemisch. Während der Reaktion beträgt das pH $<1$ und die Reaktionstemperatur 48 °C. Das Reaktionsgemisch wird abgekühlt und filtriert. Man erhält 889 Teile (99,4% der Theorie) Diäthylentriaminopentaacetonitril vom Fp 104 bis 105 °C. GC-Reinheit $>99,8$ Gewichtsprozent.

Beispiel 4 (Vergleich)

In einem Rührkessel werden 450 Teile 37-prozentiger wässriger Formaldehyd (5,55 Mole) und 132 Teile Ammoniumsulfat (2 Mole $NH_4^{\oplus}$) erwärmt. Dazu leitet man aus einem kühlbaren Zulaufgefäss eine Mischung von 500 Teilen Wasser, 30,6 Teilen conc. Schwefelsäure und 167,4 Teile Blausäure innerhalb von 15 Minuten ein. Nach beendetem Zulauf liegt ein pH-Wert von 0,51 vor und die Temperatur steigt auf ca. 70 °C, wobei ein Blausäure-Rückfluss beobachtet wird. Die Blausäurekonzentration während der Reaktion beträgt 4,56 Prozent. Nach dem Abkühlen des Reaktionsgemisches auf 25 °C wird der Feststoff abfiltriert und bei 80 °C getrocknet. Man erhält eine Ausbeute von 66,42% der Theorie an Nitrilotriacetonitril vom Fp 127,5 °C und eine GC-Reinheit von 96,4 Prozent.

Beispiel 5 (Vergleich)

In einem Rührbehälter wurden unter kräftigem Rühren bei 10 °C 53,5 Teile Ammoniumchlorid und 255 Teile wässriger, 37 Gew.-%iger Formaldehyd vorgelegt und mit wenigen Tropfen HCl conz. auf pH 1,2 eingestellt. Dazu gab man langsam innerhalb einer halben Stunde 89 Teile Blausäure. Die Konzentration der Blausäure wurde mit der Blausäurebestimmung nach Liebig gemessen. Nach beendeter Blausäurezugabe zeigt eine Probe des Reaktionsgemisches 19% Blausäure, bezogen auf das Reaktionsgemisch.

Nach weiteren 20 Minuten betrug die Blausäurekonzentration 18,2%. Danach wurde das Reaktionsgemisch auf 45 bis 90 °C erwärmt. Nach 3 Stunden war die Blausäure-Konzentration noch 9,45%, nach 4 Stunden 3,3% Blausäure. Danach wurde das Gemisch auf 15 °C abgekühlt und abfiltriert.

Man erhielt 93,5 Teile (69,8% der Theorie) Nitrilotriacetonitril (NTAN) mit einem Fp. 124,5 bis 126,5 °C.

**Patentanspruch**

Verfahren zur Herstellung von Aminoacetonitrilen der Formel

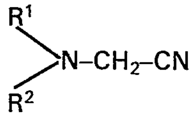

I,

worin $R^1$ und $R^2$ gleich oder verschieden sein können und jeweils den Rest $-CH_2-CN$ oder den Rest

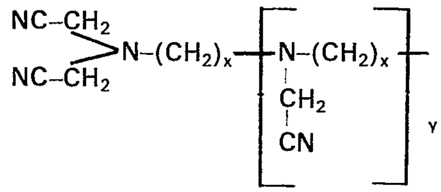

bedeuten, $R^1$ auch für einen aliphatischen Rest steht, die einzelnen Reste x und y gleich oder verschieden sein können und jeweils eine ganze Zahl bezeichnen, y auch für 0 steht, durch Umsetzung von Ammoniak oder Aminen mit Formaldehyd und Blausäure in Gegenwart einer zusätzlichen Säure, dadurch gekennzeichnet, dass man Stickstoffverbindungen der Formel

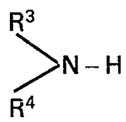

II,

worin $R^3$ und $R^4$ ein Wasserstoffatom bedeuten, $R^3$ und/oder $R^4$, auch, wenn $R^1$ und/oder $R^2$ den Rest

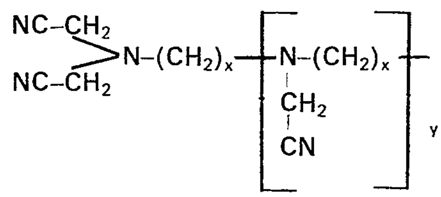

bezeichnen, für den Rest

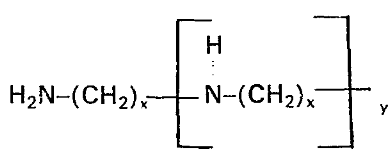

stehen,

$R^3$ auch, wenn $R^1$ für einen aliphatischen Rest steht, die Bedeutung von $R^1$ besitzt, x und y die vorgenannte Bedeutung besitzen, bei einem pH unterhalb 2 bei einer Temperatur von 10 bis 70 °C umsetzt, wobei die Konzentration der Blausäure während der Reaktion nicht mehr als 3 Gewichtsprozent, bezogen auf das Reaktionsgemisch, beträgt.

**Revendication**

Procédé pour la préparation d'aminoacétonitriles de formule

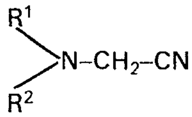

I,

dans laquelle $R^1$ et $R^2$ peuvent être semblables ou différents et représentent chacun le radical $-CH_2-CN$ ou le radical

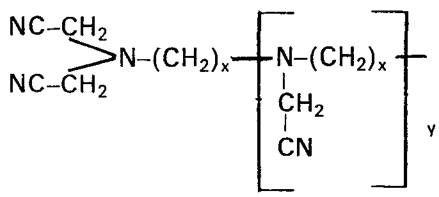

$R^1$ pouvant être également mis pour

un radical aliphatique, les différents symboles x et y pouvant êtres égaux ou différents et représentant chacun un nombre entier, y pouvant être mis pour 0, par réacetion d'ammoniac ou d'amines avec le formaldéhyde et l'acide cyanhydrique en présence d'un acide supplémentaire, caractérisé en ce qu'on fait réagir des composés azotés de formule

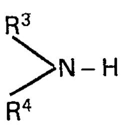

II,

dans laquelle $R^3$ et $R^4$ représentent l'un et l'autre un atome d'hydrogène, $R^3$ et/ ou $R^4$ pouvant être également mis pour le radical

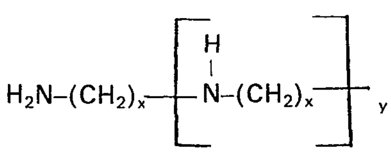

lorsque $R^1$ et/ou $R^2$ représentent le radical

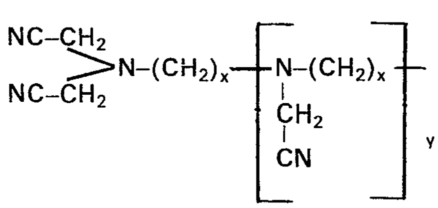

$R^3$ pouvant aussi avoir la signification de $R^1$ lorsque $R^1$ est mis pour un radical aliphatique, x et y ayant la signification donnée ci-dessus, à un pH de moins de 2 et à une température de 10 à 70 °C, la concentration de l'acide cyanhydrique pendant la réaction ne s'élevant pas à plus de 3% en poids par rapport au mélange réactionnel.

**Claim**

A process for the preparation of an aminoacetonitrile of the formula

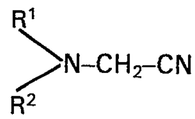

I,

where $R^1$ and $R^2$ may be identical or different and each is $-CH_2-CN$ or

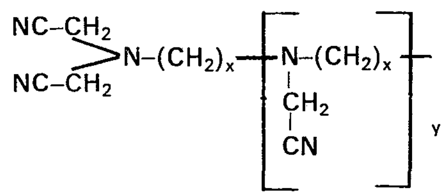

$R^1$ can also be an aliphatic radical and the symbols x and y can be identical or different and each is an integer, and y can also be 0, by reacting ammonia or an amine with formaldehyde and hydrogen cyanide in the presence of an additional acid, wherein a nitrogen compound of the formula

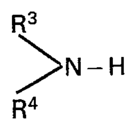

II,

where $R^3$ and $R^4$ are each hydrogen, or $R^3$ and/or $R^4$ are

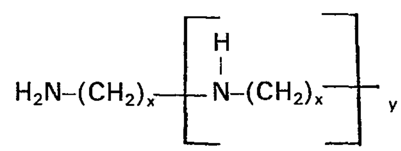

if $R^1$ and/or $R^2$ are

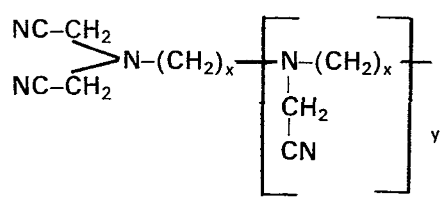

and $R^3$ can also have the same meaning as $R^1$ if $R^1$ is an aliphatic radical, and x and y have the above meanings, is reacted at a pH of below 2 at a temperature of from 10 to 70 °C, the concentration of hydrogen cyanide during the reaction being not more than 3 per cent by weight, based on the reaction mixture.